(19) Europäisches Patentamt
European Patent Office
Office européen des brevets

(11) Veröffentlichungsnummer: **0 189 129**
**A2**

# (12) EUROPÄISCHE PATENTANMELDUNG

(21) Anmeldenummer: **86100541.1**

(22) Anmeldetag: **17.01.86**

(51) Int. Cl.⁴: **C 07 C 69/34**, C 07 C 31/125, C 07 D 307/20, C 07 C 33/025, C 07 C 69/612, C 07 C 69/708

(30) Priorität: **23.01.85 DE 3502079**

(43) Veröffentlichungstag der Anmeldung: **30.07.86 Patentblatt 86/31**

(84) Benannte Vertragsstaaten: **BE DE FR GB IT NL**

(71) Anmelder: **BASF Aktiengesellschaft, Carl-Bosch-Strasse 38, D-6700 Ludwigshafen (DE)**

(72) Erfinder: **Menig, Helmuth, Dr., Hauptstrasse 22, D-6701 Friedelsheim (DE)**
Erfinder: **Halbritter, Klaus, Dr., Schwarzwaldstrasse 19, D-6800 Mannheim 1 (DE)**

## (54) Verfahren zur Addition von Wasserstoff und organischen Resten an olefinisch und acetylenisch ungesättigte Verbindungen.

(57) Addition von H und einem Rest I'

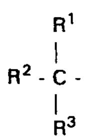

I'

($R^1$ = org. C-Rest; $R^2$ = H, $R^1$; $R^3$ = $C_1$-$C_4$-Alkyl) an olefinisch oder acetylenisch ungesättigte Verbindungen II

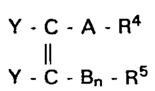

II

(Y = H oder zusammen eine weitere Bindung; n = 0 oder 1; A, B = -CO-O-; -O-CO-; -CRR-O-; -CR-NR-; R = H, $C_1$-$C_4$-Alkyl, gleich oder verschieden; $R^4$, $R^5$ = H, org. Rest; A + B (n = 1) bzw. A + B (n = 0) können zu 5- oder 6-Ring verbunden sein) unter Bildung von IIIa und IIIb

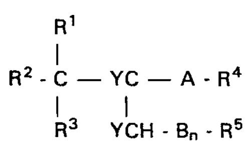

IIIa

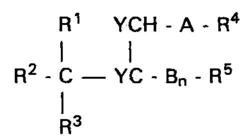

IIIb

in dem man Aldehyde I

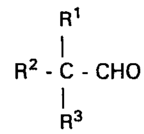

I

mit II sowie organischen Radikalbildnern umsetzt.

EP 0 189 129 A2

# Verfahren zur Addition von Wasserstoff und organischen Resten an olefinisch und acetylenisch ungesättigte Verbindungen

Die vorliegende Erfindung betrifft ein neues Verfahren zur Addition von Wasserstoff und organischen Resten der allgemeinen Formel I'

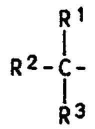

I'

in welcher $R^1$ seinerseits einen organischen C-Rest, $R^2$ Wasserstoff oder einen organischen C-Rest und $R^3$ eine $C_1$-$C_4$-Alkylgruppe bedeuten, an olefinisch oder acetylenisch ungesättigte Verbindungen der allgemeinen Formel II

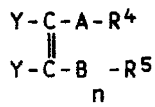

II

in der die Reste Y für Wasserstoff oder gemeinsam für eine weitere Bindung stehen, in der A und B die Gruppierungen

-CO-O-
-O-CO-
-CRR-O- oder
-CRR-NR-

bedeuten, wobei n für null oder 1 steht und wobei A und B oder A und $R^5$ auch zu einem 5- oder 6-gliedrigen Ring verbunden sein können, in der $R^4$ und die Reste $R^5$ Wasserstoff oder organische Reste und die Reste R Wasserstoff oder gleiche oder verschiedene $C_1$-$C_4$-Alkylgruppen bedeuten, unter Bildung der Additionsprodukte der allgemeinen Formel IIIa und IIIb

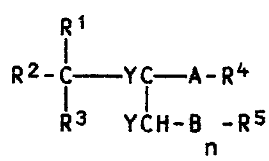

IIIa

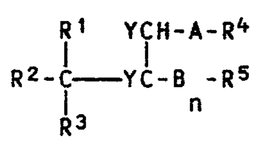

IIIb

Mi/P

Aus Organic Reactions, Band 13, Seiten 91-141 ist es bekannt, Wasserstoff und organische Reste verschiedener Art unter C-C-Verknüpfung radikalisch, also mittels Radikalbildnern wie Peroxiden oder Gammastrahlung an olefinisch oder acetylenisch ungesättigte Verbindungen zu addieren.

Handelt es sich bei den zu addierenden Verbindungen um Aldehyde R'-CHO, so erhält man in allen bisher bekannt gewordenen Fällen (s. Table IX, Seite 132 loc. cit.) nach dem Schema

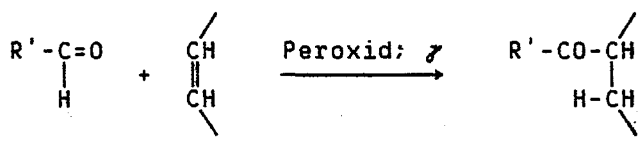


Ketone als Additionsprodukte.

In den Erläuterungen auf Seite 108 loc. cit. heißt es hierzu, daß in einigen Fällen, z.B. im Falle des Trimethylacetaldehydes (Pivalaldehyd) oberhalb 100°C auch Kohlenmonoxid freigesetzt werden könne und daß man durch Wahl geeigneter Reaktionsbedingungen auch Kohlenwasserstoffe synthetisieren könne, jedoch fehlt jede nähere Angabe über die allgemeine Art der Reaktionspartner sowie über die Bedingungen einer derartigen Reaktion.

Der Erfindung lag nun die Aufgabe zugrunde, die präparative Technik um eine neue Methodik zur Addition von Wasserstoff und organischen Resten unter C-C-Verknüpfung zu bereichern.

Demgemäß wurde gefunden, daß man Wasserstoff und die eingangs definierten Reste I' an die ebenfalls eingangs definierten olefinisch oder acetylenisch ungesättigten Verbindungen II unter Bildung der Additionsprodukte IIIa und IIIb anlagern kann, wenn man Aldehyde der allgemeinen Formel I

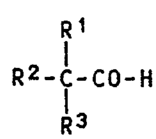
I

mit den Verbindungen II sowie mit organischen Radikalbildnern (IV) umsetzt.

Hinsichtlich der Aldehyde I ist der Verzweigungsgrad am $\alpha$-C-Atom von entscheidendem Einfluß, d.h. die erfindungsgemäße Reaktion gelingt am besten, wenn $R^2 \neq H$ ist. Für den Fall $R^2 = H$ ist dagegen mit vergleichsweise geringeren Ausbeuten zu rechnen.

Nach den bisherigen Beobachtungen hat die Art der organischen Reste $R^1$-$R^3$ prinzipiell keinen Einfluß auf die erfindungsgemäße Umsetzung; allenfalls können bestimmte Substituenten wie z.B. Hydroxylgruppen zu Nebenreaktionen Anlaß geben.

Bevorzugte Reste $R^1$ sind Alkylgruppen mit 1 bis 20 C-Atomen, vor allem die Methylgruppe, Cycloalkylgruppen wie die Cyclohexylgruppe, Arylgruppen wie die Phenylgruppe und Aralkylgruppen wie die Benzylgruppe. $R^2$ ist vorzugsweise Wasserstoff oder eine $C_1$-$C_4$-Alkylgruppe, insbesondere die Methylgruppe, und $R^3$ bedeutet vorzugsweise die Methylgruppe.

Diese Reste können ihrerseits substituiert sein, z.B. durch Chlor, Alkoxygruppen wie die Methoxygruppe sowie durch Carbalkoxygruppen.

Die meisten der Aldehyde I sind ihrerseits durch Hydroformylierung der entsprechenden Olefine $R^{1'}=CR^2R^3$ zugänglich. Besondere Bedeutung hat das erfindungsgemäße Verfahren im Falle des Pivalaldehydes ($R^1=R^2=R^3=CH_3$), d.h. zur Addition der tert.-Butylgruppe an die Doppel- bzw. Dreifachbindung von II.

Darauf, daß die olefinisch bzw. acetylenisch ungesättigten Verbindungen II im erfindungsgemäßen Sinne reagieren, haben die definitionsgemäßen aktivierenden Gruppierungen A bzw. A und B einen wesentlichen Einfluß. Auf die Reste R, welche vorzugsweise Wasserstoff bedeuten, kommt es hingegen nicht entscheidend an.

Auch die Reste $R^4$ und $R^5$ können prinzipiell beliebig sein, da sich deren Art nur noch geringfügig auf das Reaktionsgeschehen an der Mehrfachbindung auswirkt. Bevorzugt werden jedoch Wasserstoff und Kohlenwasserstoffreste mit 1 bis 20 C-Atomen.

Besonders gut gelingt die Umsetzung, wenn beide C-Atome der Mehrfachbindung durch die Gruppierungen A und B (n = 1) aktiviert sind und wenn A und B bzw. A und $R^5$ zu einem 5- oder 6-gliedrigen Ring verbunden sind, also gemeinsam z.B. folgende Gruppierungen bilden:

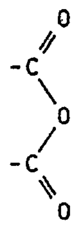 , 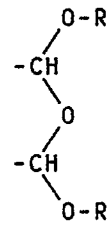 , 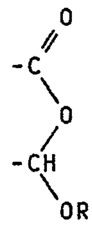 und 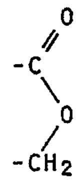

Handelt es sich um symmetrische Verbindungen II, erhält man einheitliche Verfahrensprodukte III (IIIa = IIIb), und sind die Substituenten $-A-R^4$ und $-(B)_n-R^5$ verschieden, können die beiden isomeren Verbindungen IIIa und IIIb entstehen, jedoch ist die Bildung eines dieser Isomeren meist bevorzugt.

Als Beispiele für die Ausgangsverbindungen II seien genannt:

- Acrylsäure, Crotonsäure, Zimtsäure und Propargylsäure sowie deren Ester, z.B. die Ethylester;
- Fumarsäure und Maleinsäure sowie deren Bisester, z.B. die Bisethylester;
- Maleinsäureanhydrid;
- Vinylester wie Vinylacetat;
- Allylalkohol, Propargylalkohol, Buten-1,4-diol und Butin-1,4-diol sowie deren Ether und Ester wie die Methylether und die Acetate;
- 3-Methylbut-1-en-3-ol und 3-Methylbut-1-in-3-ol;
- 2,5-Dimethoxydihydrofuran, 2-Methoxydihydrofuran-5-on, 2,5-Dihydrofuran-2-on und 2-Methoxy-2,5-dihydrofuran und
- 3,6-Dimethoxycyclohex-1-en.

Als Radikalbildner (IV) kommen in erster Linie organische Peroxide wie Hydroperoxide (R-O-O-H) und Peroxide (R-O-O-R') in Betracht, die im Bereich der Reaktionstemperaturen mit merklicher Geschwindigkeit in Radikale zerfallen, z.B. tert.-Butylhydroperoxid, Cumolhydroperoxid, Di-tert.-butylperoxid, Dicumylperoxid, Dibenzoylperoxid und tert.-Butylper-2-ethylhexanoat, daneben aber auch sonstige Radikalbildner wie beispielsweise Azodiisobutyronitril.

Da die Umsetzung über einen Radikalkettenmechanismus verläuft, genügen meistens relativ geringe Mengen an den Radikalbildnern, und zwar etwa 0,1-20, vorzugsweise 1-10 mol.% IV pro Mol des Aldehydes I.

Den Aldehyd I und die ungesättigte Verbindung setzt man zweckmäßigerweise im Verhältnis 1:1 bis 10:1, vorzugsweise 2:1 bis 6:1 ein.

Die Umsetzung findet im allgemeinen zwischen 50 und 200°C, vorzugsweise zwischen 70 und 150°C statt. Im Einzelfall wählt man zweckmäßigerweise eine Temperatur, bei der die Zerfallshalbwertszeit des Radikalbildners etwa 1 h, aber nicht wesentlich weniger, beträgt, um unerwünschte Nebenreaktionen, vor allem die Polymerisation von II, nach Möglichkeit zu unterdrücken.

Der Polymerisation läßt sich auch dadurch entgegenwirken, daß man den Aldehyd I bei Reaktionstemperatur vorlegt und diesen mit einem Gemisch aus II und IV versetzt. Im Moment der Zugabe zerfällt der Radikalbildner, so daß die meist stark bevorzugte erfindungsgemäße Reaktion sofort einsetzen kann. Um ein Überangebot an Radikalen zu vermeiden, welches zu Nebenreaktionen wie der Polymerisation oder auch zu Radikalkombinationen führen kann, empfiehlt sich eine allmähliche, auf einige Stunden verteilte Zugabe des Gemisches aus II und IV.

Man kann in Gegenwart eines inerten Lösungsmittels, z.B. von aliphatischen oder aromatischen Kohlenwasserstoffen arbeiten, jedoch ist dies meistens nur erforderlich, wenn einer der Reaktionspartner unter den Reaktionsbedingungen nicht flüssig ist.

Die Aufarbeitung der Reaktionsgemische kann durch Destillation und/oder Kristallisation wie üblich erfolgen, so daß nähere Angaben sich hierzu erübrigen.

Das erfindungsgemäße Verfahren stellt eine wertvolle Bereicherung der präparativen Technik dar, indem es die Addition verzweigter organischer Reste an die Mehrfachbedingung bestimmter ungesättigter Verbindungen unter C-C-Verknüpfung gestattet. Dies hat vor allem für die Einführung der tert.-Butylgruppe, ausgehend von Pivalaldehyd, Bedeutung, da diese Gruppe häufig ein charakteristisches Strukturelement für physiologische Wirkstoffe und Antioxidantien darstellt.

Beispiele 1 - 12

Je 2,78 mol eines Aldehydes I wurden auf 75°C erhitzt und bei dieser Temperatur im Laufe von $t_1$ Stunden mit einem Gemisch aus 0,556 mol einer ungesättigten Verbindung II und 0,0556 mol tert.-Butylper-2-ethylhexanoat versetzt. Anschließend wurde das Gemisch noch $t_2$ weitere Stunden auf 75°C gehalten.

Die Einzelheiten dieser Versuche sowie deren Ergebnisse sind der nachstehenden Tabelle zu entnehmen.

Tabelle. Beispiele 1-12

| Beisp. Nr. | I | II | $t_1$ [h] | $t_2$ [h] | Umsatz 1) [%] | III | Ausbeute 2) [%] | Sdp. [°C/mbar] |
|---|---|---|---|---|---|---|---|---|
| 1 | Pivalaldehyd | Maleinsäurediethylester | 1,8 | 5 | 100 | tert.-Butylbernsteinsäurediethylester | 85 | 69/0,35 |
| 2 | Pivalaldehyd | 3-Methylbut-1-en-3-ol | 1,8 | 5 | 40 | 2,5,5-Trimethyl-hexan-2-ol | 33 | 33/0,2 |
| 3 | Pivalaldehyd | 3-Methylbut-1-in-3-ol | 1,8 | 5 | 60 | 2,5,5-Trimethyl-hex-3-en-2-ol | 48 | 27/0,2 |
| 4 | Pivalaldehyd | Maleinsäureanhydrid | 1,8 | 5 | 100 | tert.-Butylbernsteinsäureanhydrid | 65 | 90/1,0 |
| 5 | Pivalaldehyd | 2,5-Dimethoxy-dihydrofuran | 1,8 | 5 | 50 | 2,5-Dimethoxy-3-tert.-butyltetrahydrofuran | 37 | 30-35/0,2 |
| 6 | Pivalaldehyd | 2-Methoxy-2,5-dihydrofuran-5-on | 1,8 | 5 | 100 | 2-Methoxy-3-tert.-butyl-tetrahydrofuran-2-on | 70 | 70/0,5 |
| 7 | 2-Phenylpropanal | Maleinsäurediethylester | 1,8 | 5 | 10 | 3,5-Diethoxycarbonyl-2-phenylbutan | 6 | nicht bestimmt |
| 8 | Pivalaldehyd 3) | Maleinsäurediethylester | 2 | 5 | 100 | tert.-Butylbernsteinsäurediethylester | 83 | 69/0,35 |
| 9 | n-Butoxypivalaldehyd | Maleinsäurediethylester | 2 | 5 | 90 | (n-Butoxy-tert.-butyl)-bernsteinsäurediethylester | 69 | 96/0,2 |
| 10 | Pivalaldehyd | Fumarsäurediethylester | 2 | 5 | 100 | tert.-Butylbernsteinsäurediethylester | 71 | 69/0,35 |
| 11 | iso-Butyraldehyd | Maleinsäurediethylester | 2 | 20 | 90 | iso-Propylbernsteinsäurediethylester | 41 | 90/0,5 |
| 12 | 2-Methylbutanal | Maleinsäurediethylester | 2 | 16 | 100 | n-But-2-yl-bernsteinsäurediethylester | 38 | 85/0,3 |

1) bezogen auf II
2) bezogen auf eingesetztes II
3) mit Azodiisobutyronitril anstelle des Peroxides

Patentansprüche

1. Verfahren zur Addition von Wasserstoff und organischen Resten der allgemeinen Formel I'

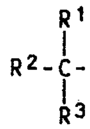
I'

in welcher $R^1$ seinerseits einen organischen C-Rest, $R^2$ Wasserstoff oder einen organischen C-Rest und $R^3$ eine $C_1$-$C_4$-Alklylgruppe bedeuten, an olefinisch oder acetylenisch ungesättigte Verbindungen der allgemeinen Formel II

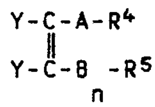
II

in der die Reste Y für Wasserstoff oder gemeinsam für eine weitere Bindung stehen, in der A und B die Gruppierungen

-CO-O-
-O-CO-
-CRR-O- oder
-CRR-NR-

bedeuten, wobei n für null oder 1 steht und wobei A und B oder A und $R^5$ auch zu einem 5- oder 6-gliedrigen Ring verbunden sein können, in der $R^4$ und die Reste $R^5$ Wasserstoff oder organische Reste und die Reste R Wasserstoff oder gleiche oder verschiedene $C_1$-$C_4$-Alkylgruppen bedeuten, unter Bildung der Additionsprodukte der allgemeinen Formel IIIa und IIIb

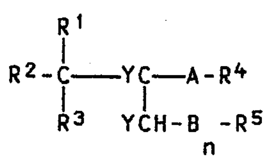
IIIa

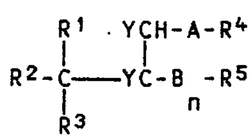
IIIb

dadurch gekennzeichnet, daß man Aldehyde der allgemeinen Formel I

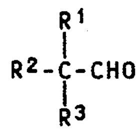

I

mit den Verbindungen II sowie mit organischen Radikalbildnern (IV) umsetzt.

2. Verfahren nach Anspruch 1, dadurch gekennzeichnet, daß man als Aldehyd I Pivalaldehyd ($R^1=R^2=R^3=CH_3$) einsetzt.

3. Verfahren nach den Ansprüchen 1 und 2, dadurch gekennzeichnet, daß die Reste $R^4$ und $R^5$ $C_1$-$C_4$-Alkylgruppen und die Reste R in den Verbindungen II Wasserstoff bedeuten.

4. Verfahren nach den Ansprüchen 1 und 2, dadurch gekennzeichnet, daß n in den Verbindungen II den Wert 1 hat.

5. Verfahren nach den Ansprüchen 1 und 2, dadurch gekennzeichnet, daß die Gruppierungen A und B (n = 1) bzw. A und $R^5$ (n = 0) in den Verbindungen II zu einem 5- oder 6-gliedrigen Ring verbunden sind.

6. Verfahren nach den Ansprüchen 1 bis 5, dadurch gekennzeichnet, daß die Reaktionstemperaturen zwischen 50 und 200°C liegen.